(19) Europäisches Patentamt
European Patent Office
Office européen des brevets

(11) **EP 1 379 845 B1**

(12) **EUROPÄISCHE PATENTSCHRIFT**

(45) Veröffentlichungstag und Bekanntmachung des Hinweises auf die Patenterteilung:
**15.08.2007 Patentblatt 2007/33**

(21) Anmeldenummer: **02742863.0**

(22) Anmeldetag: **21.03.2002**

(51) Int Cl.:
***G01J 3/00*** *(2006.01)*

(86) Internationale Anmeldenummer:
**PCT/EP2002/003182**

(87) Internationale Veröffentlichungsnummer:
**WO 2002/079738 (10.10.2002 Gazette 2002/41)**

(54) **VORRICHTUNG ZUR SIMULTANEN DETEKTION VON STRAHLUNGEN UNTERSCHIEDLICHER WELLENLÄNGE**

DEVICE FOR THE SIMULTANEOUS DETECTION OF RADIATION OF DIFFERENT WAVELENGTH

DISPOSITIF DE DETECTION SIMULTANEE DE RAYONNEMENTS DE LONGUEURS D'ONDES DIFFERENTES

(84) Benannte Vertragsstaaten:
**AT BE CH CY DE DK ES FI FR GB GR IE IT LI LU MC NL PT SE TR**

(30) Priorität: **22.03.2001 DE 10114748**

(43) Veröffentlichungstag der Anmeldung:
**14.01.2004 Patentblatt 2004/03**

(73) Patentinhaber: **ESE Embedded System Engineering GmbH**
**78333 Stockach (DE)**

(72) Erfinder: **HABERSTROH, Klaus**
**78351 Bodman-Ludwigshafen (DE)**

(74) Vertreter: **Hössle Kudlek & Partner Patentanwälte,**
**Postfach 10 23 38**
**70019 Stuttgart (DE)**

(56) Entgegenhaltungen:
**EP-A- 0 845 238          EP-A- 0 994 342**
**WO-A-94/04893          DE-A- 3 406 175**
**DE-A- 3 818 278          DE-A- 4 425 636**
**US-A- 4 880 304          US-A- 6 157 454**

**Beschreibung**

Die Erfindung betrifft eine Vorrichtung zur simultanen bzw. gleichzeitigen Detektion von Strahlungen unterschiedlicher Wellenlänge. Solche Vorrichtungen werden bspw. zur Messung von fluoreszierenden Farbstoffen im menschlichen Auge eingesetzt. Aus den Fluoreszenzmessungen können direkt die Konzentrationen körpereigener Stoffe abgeleitet werden. Außerdem betrifft die Erfindung eine Akkuladeeinheit für eine derartige Vorrichtung und ein optomechanisches Basismodul zum modularen Aufbau einer derartigen Meßvorrichtung.

Durch Detektion der Strahlung kann auf die Konzentration des die bestimmte Strahlung emittierenden Stoffes rückgeschlossen werden.

Es ist bekannt, die Konzentration bestimmter körpereigener Stoffe bzw. Zusammensetzungen aus der Augen- bzw. Tränenflüssigkeit nachzuweisen bzw. (quantitativ) zu bestimmen. Ein Beispiel hierfür ist die Bestimmung des Glucosegehaltes im Körper mittels eines Fluoreszenz-basierenden Verfahrens. Hierbei sind zwei fluoreszierende Substanzen in das menschliche Auge eingebracht, beispielsweise mittels Kontaktlinsen, die diese Substanzen enthalten. Eine erste Substanz dient dabei zur Messung der Konzentration des zu messenden körpereigenen Stoffes, wie beispielsweise von Glucose. Die Energie des emittierten Lichts der ersten Substanz ist abhängig von der Konzentration der körpereigenen Stoffe in der Tränenflüssigkeit. Die zweite Substanz dient zur Referenzmessung. Die Energie des emittierten Lichts der zweiten Substanz ist unabhängig von der Konzentration des zu messenden körpereigenen Stoffes. Die Referenzmessung ermöglicht es, Schwankungen der Anregungsenergie und des Abstandes zwischen dem Sensor und dem Auge zu eliminieren. Andere einsetzbare Beispiele von biochemischen Detektionsverfahren sind beispielsweise Lumineszenz, Surface Plasma Resonance (SPR), Reflexion etc.

Das bekannte Verfahren wurde mit Standardsensoren und externen Lichtquellen in Laborumgebung durchgeführt bzw. verifiziert. Die Verifizierung erfordert allerdings einen komplexen Versuchsaufbau. Um reproduzierbare Ergebnisse zu erhalten wurde die Messung mit konstanten Umgebungsbedingungen durchgeführt.

Die im Labor verwendete Vorrichtung ist für eine Verwendung durch einen Patienten im täglichen Gebrauch nicht geeignet, da diese nicht nur zu teuer und zu groß (Laboraufbau), sondern auch nur unter definierten Rahmenbedingungen und von Fachkräften betreibbar ist.

Demgegenüber wird eine Vorrichtung zur simultanen Detektion von Strahlungen unterschiedlicher Wellenlänge mit einer Anzahl von übereinander angeordneten Basismodulen, einem Optikmodul mit einem Objektiv und einem Elektronikmodul mit lichtdetektierenden Elementen vorgestellt. In den Basismodulen ist jeweils eine Einrichtung zum Reflektieren bzw. Umleiten von Strahlung eines vorbestimmbaren Wellenlängenbereichs vorgesehen. Die lichtdetektierenden Elemente korrespondieren jeweils mit einer der Einrichtungen, d.h. daß die Einrichtungen Strahlungen vorbestimmter Wellenlängenbereiche so umleiten, daß diese auf die entsprechenden lichtdetektierenden Element treffen. Sind außerdem lichtemittierende Elemente vorgesehen, korrespondieren diese entsprechend mit den jeweiligen Einrichtungen.

Die erfindungsgemäße Vorrichtung ist vorzugsweise eine handgehaltene Vorrichtung zur Messung körpereigener Stoffe durch optische Detektion im menschlichen Auge, die derart klein und handlich und zudem einfach in der Bedienung ist, daß sie von Patienten ohne fachliche Anleitung zur Auto-Diagnose selbst verwendet werden kann.

Die erfindungsgemäße Vorrichtung kann aber auch bspw. in der Prozeßkontrolle eingesetzt werden.

Die Erfindung eignet sich insbesondere zur Anwendung im Zusammenhang mit einem vorstehend geschilderten Fluoreszenzbasierten Verfahren zur Messung der körpereigenen Glucosekonzentration, ist jedoch gleichermaßen zur Verwendung von Messungen geeignet, mittels derer andere Stoffe (wie Lactat u.a. in der Tränenflüssigkeit enthaltene Stoffe) bestimmt werden.

Das Meßgerät kann gleichzeitig reflektiv Fluoreszenzstoffe in beliebigen Materialien, wie bspw. Weißmacher in der Papierfertigung, messen.

In Ausgestaltung der Erfindung ist eine Anordnung von jeweils drei Spiegeln und dichroitischen Strahlteilern oder drei dichroitische Strahlteiler-Paare vorgesehen, die einen konfokalen Strahlengang der Referenzwellenlänge und der Meßwellenlänge bewirken. Dadurch wird erreicht, daß die Meßwerte unempfindlich gegenüber Veränderungen des Meßwinkels sind. Des weiteren erfolgt die Ansteuerung der verwendeten Lichtquellen, bei denen es sich vorteilhafterweise um Leuchtdioden handelt, getaktet, wodurch das Umgebungslicht bzw. Streulicht ausgefiltert werden kann.

Der Aufbau der erfindungsgemäßen Vorrichtung ist modular, wodurch eine kostengünstige Massenfertigung ermöglicht wird. Das Grundmodul ist hierbei vorzugsweise eine mit Bohrungen und Ausnehmungen versehene Platte. Die Bohrungen und Ausnehmungen sind in vorbestimmten Winkelabständen zueinander im wesentlichen rotationssymmetrisch angeordnet. In die Ausnehmungen sind die Spiegel und Strahlteiler über den Bohrungen eingesetzt, wobei in einer Platte jeweils ein Strahlteiler-Paar (zwei identische Strahlteiler) oder ein Strahlteiler und ein Spiegel eingesetzt sind. Drei derartig ausgerüstete Platten sind bspw. zur Bildung der erfindungsgemäßen Vorrichtung derart aufeinandergesetzt, daß durch die einzelnen Bohrungen in den Platten Kanäle für die einzelnen Strahlengänge entstehen, die in einem einem Objektiv zugewandten Abschnitt konfokal verlaufen und in einem dem Objektiv abgewandten Abschnitt aufgetrennt in verschiedenen Kanälen verlaufen. In dem dem Objektiv abgewandten Abschnitt sind jedem Kanal entweder lichtemittierende oder - detektierende Elemente angeordnet. Die Spiegel und Strahlteiler sind derart angeordnet, daß ein von einem lichtemittierenden Element ausgesandter Lichtstrahl in einen konfokalen Strahlengang bzw. den zugeordneten Kanal reflektiert wird und aus der Richtung des Auges kommendes Licht (von den Substanzen im Auge bzw. in der Tränenflüssigkeit emittiertes Licht) in einen Strahlengang bzw. Kanal reflektiert wird, an dessen anderem Ende sich das zugeordnete Detektorelement befindet.

Die Anregungslichtquellen werden vorteilhafterweise moduliert, so daß das Nutzsignal vom Signal des Umgebungslichts ausgefiltert werden kann.

Insbesondere bei Fluoreszenz-basierender Detektion sind die Anregungslichtquellen und die Meßsensoren der verwendeten Anregungssubstanzen konfokal im Strahlengang angeordnet. Der Strahlengang ist so konstruiert, daß der Abstand zwischen den Anregungslichtquellen und dem Auge sowie der Abstand zwischen den Sensoren und dem Auge identisch sind. Somit ist für alle Strahlengänge nur eine optische Abbildung (beispielsweise durch ein Objektiv) erforderlich.

Die Tiefenschärfe des abbildenden Objektivs bestimmt, wie empfindlich sich das Meßgerät bezüglich des Abstandes zwischen Meßgerät und Objekt bzw. Auge verhält. Bei einem Handmeßgerät wird eine geringe Tiefenschärfe gewählt, so daß der Abstand Auge-Meßgerät einen geringen Einfluß auf das Meßgerät hat.

Des weiteren wird erfindungsgemäß eine Akkuladeeinheit für die handgehaltene und batteriebetriebene Vorrichtung der Erfindung bereitgestellt. Diese Ladeeinheit zeichnet sich dadurch aus, daß aus der Messung abgeleitete und in einer Speichereinrichtung des Handmeßgerätes gespeicherte Daten während eines Ladevorgangs des Handmeßgerätes über eine in der Ladeeinheit enthaltene Kommunikationsschnittstelle an einen Empfänger übertragen werden können. Bei dem Empfänger handelt es sich insbesondere um ein Datenverarbeitungssystem in Form eines Personal Computers, eines digitalen Datenverwalters (PDA - Personal Digital Assistent) o.ä.

Die Analogelektronik, die Meßwerterfassung und die Speicherung der Meßdaten sind vorteilhafterweise in das Handgerät integriert. Die Stromversorgung für diese Funktionen erfolgt vorzugsweise durch eine aufladbare Batterie.

Die Erfindung stellt somit eine handgehaltene Meßvorrichtung bereit, die

- als Handmeßgerät mit Batteriebetrieb verwendbar ist, d.h.
- einen kompakten optischen Aufbau und
- geringen Stromverbrauch (Batteriebetrieb) aufweist
- mit einer für das Auge verträglichen Lichtenergie (Wellenlänge und Intensität) anregt
- unempfindlich gegenüber Änderungen des Abstands zum Auge reproduzierbare Meßergebnisse liefert
- unempfindlich gegenüber Änderungen des Umgebungslichts reproduzierbare Meßergebnisse liefert
- unempfindlich gegenüber Änderungen des Winkels zum Auge reproduzierbare Meßergebnisse liefert
- kostengünstig in der Produktion ist, d.h.
- fertigungstechnisch so aufgebaut ist, daß die Montage in großen Stückzahlen automatisiert erfolgen kann, und
- keine aufwendige optische Justage erfordert
- einfach in der Handhabung ist
- eine standardisierte Schnittstelle zur Speicherung und Verwaltung der Meßergebnisse aufweist.

Die mit der Erfindung verbundenen Vorteile sind insbesondere:

a. Die Erfindung ermöglicht die Herstellung eines kostengünstigen Handmeßgerätes zur Messung von körpereigenen Stoffen in der Tränenflüssigkeit durch optische Detektion im menschlichen Auge.

b. Die Erfindung ist optimiert für die Messung der Konzentration eines definierten körpereigenen Stoffes.

c. Die Optik ist modular aus mechanisch identischen einfachen Teilen aufgebaut.

d. Es ist keine Justage der Optik erforderlich.

e. Eine separate Lade/Kommunikationsstation dient zum Aufladen der Batterien des Handmeßgerätes und dient gleichzeitig als Kommunikationsschnittstelle zwischen dem Handmeßgerät und einem Standard-Datenerfassungsgerät.

f. Zur Kommunikation werden Standardschnittstellen verwendet. Dies kann mit einer kabelgebundenen oder kabellosen Verbindung realisiert werden.

g. Die Verwaltung der Meßdaten kann mit handelsüblichen Standard-Programmen erfolgen.

h. Die Messung ist unempfindlich gegenüber Änderungen des Abstands Meßgerät/Auge.

i. Die Messung ist unempfindlich gegenüber Änderungen des Winkels Meßgerät/Auge.

j. Es sind gleichzeitige reflektive Messungen von mehreren Fluoreszenzstoffen in der Prozeßüberwachung möglich.

Das erfindungsgemäße Verfahren zum Einstellen einer Vorrichtung sieht vor, daß eine Fokussierung durchgeführt wird. Diese kann bspw. durch Ändern des Abstands zwischen Vorrichtung und zu vermessenden Objekt durchgeführt werden, indem während des Änderns des Abstands bereits gemessen wird und anhand des Verlaufs der gemessenen Daten in Abhängigkeit des Abstands der geeignete Abstand bzw. der maximale Meßwert ermittelt wird. Der geeignete Abstand ist in der Regel der Abstand bei dem der Verlauf einen Peak aufweist.

Vorzugsweise wird im Pulsbetrieb gearbeitet. Die Pulszeit beträgt bspw. 500 ms bei einer Gesamtmeßzeit von 2 bis 3 Sekunden. Kürzere Meß- und Pulszeiten sind aber durchaus realisierbar.

Weitere Vorteile und Ausgestaltungen der Erfindung ergeben sich aus der Beschreibung und der beiliegenden Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung ist anhand eines Ausführungsbeispieles in der Zeichnung schematisch dargestellt und wird im folgenden unter Bezugnahme auf die Zeichnung ausführlich beschrieben.

Figur 1     zeigt in schematischer Darstellung eine erfindungsgemäße handgehaltene Vorrichtung zur Messung körpereigener Stoffe durch optische Detektion im menschlichen Auge (Handmeßgerät) mit einer zugehörigen Ladeeinheit mit Kommunikationsschnittstelle.

Figur 2     zeigt in stark schematischer Längsschnittdarstellung den Aufbau des erfindungsgemäßen Handmeßgerätes der Figur 1.

Figur 3     zeigt ein Diagramm mit einer Spektrendarstellung der Anregungs- und Emissionswellenlängen, die bei dem erfindungsgemäßen Handmeßgerät der Figur 2 auftreten.

Figur 4     zeigt in der Figur 2 vergleichbarer stark schematischer Längsschnittdarstellung den Aufbau des erfindungsgemäßen Handmeßgeräts der Figur 1 mit einer Veranschaulichung der vier Strahlengänge und jeweils zugeordnetem Querschnitt durch das Handmeßgerät durch die unterschiedlichen Module.

Figur 5     zeigt ein Blockschaltbild für eine Elektronik zur Ansteuerung der lichtemittierenden und -detektierenden Elemente in dem erfindungsgemäßen Handmeßgerät.

Figur 6     zeigt in perspektivischer Darstellung eine ein Basismodul des erfindungsgemäßen Handmeßgerätes bildende Platte mit Ausnehmungen und Bohrungen.

Figur 7     zeigt die Platte der Figur 6 mit eingesetztem Spiegel, Strahlteiler und Filtern.

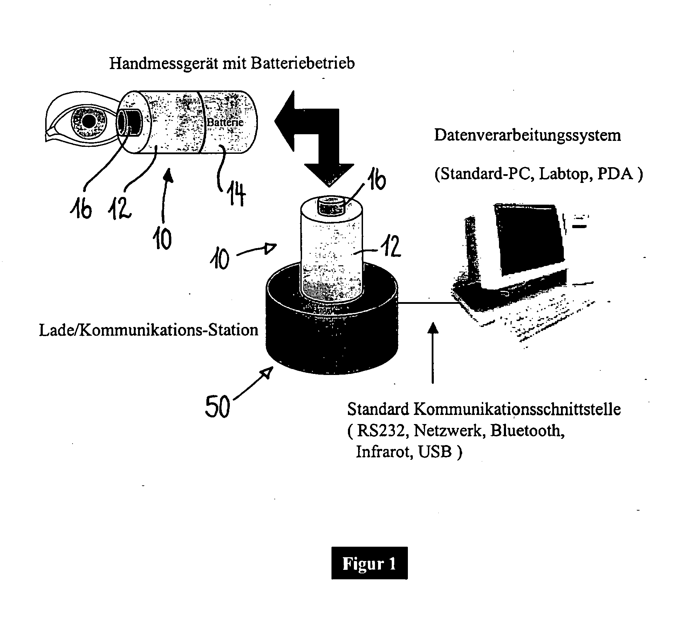

Figur 1 zeigt ein Ausführungsbeispiel einer erfindungsgemäßen handgehaltenen Vorrichtung zur Messung körpereigener Stoffe durch optische Detektion im menschlichen Auge (nachfolgend Handmeßgerät genannt). In Figur 1 ist das erfindungsgemäße Handmeßgerät 10, das battieriebetrieben ist und mittels dem eine emittierte Lichtenergie von zwei fluoreszierenden Substanzen im menschlichen Auge gemessen werden, zur Veranschaulichung dieser Messung vor ein menschliches Auge gehalten. Das Handmeßgerät 10 weist im wesentlichen zylindrische Form auf. Die Dimensionen des Handmeßgeräts 10 sind derart gewählt, daß es bequem mit der Hand gehalten werden kann. Die Anregung der Substanzen zur Durchführung der Messung erfolgt mittels zweier im Innern des Handmeßgeräts 10 angeordneter lichtemittierender Elemente (Lichtquellen) mit definierter Wellenlänge, die über ein an einem Ende des Handmeßgeräts angeordnetes Objektiv 16 aus dem Handmeßgerät austreten. Wird das Handmeßgerät vor das Auge gehalten, so trifft das Licht mit der definierten Wellenlänge auf das Auge, wo die in das Auge eingebrachten Substanzen angeregt werden. Das von den Substanzen daraufhin emittierte Licht wird über das Objektiv 16 in das Handmeßgerät geleitet und dort mittels zweier lichtdetektierender Elemente (lichtempfindliche Sensoren) detektiert. Zum Laden der Batterie des Handmeßgeräts 10 wird dieses in eine Ladeeinheit 50 gesetzt. Die Ladeeinheit verfügt erfindungsgemäß über eine Kommunikationsschnittstelle, über die während des Ladevorgangs in einer Speichereinrichtung des Handmeßgeräts gespeicherte Daten an ein Datenverarbeitungssystem übertragbar sind.

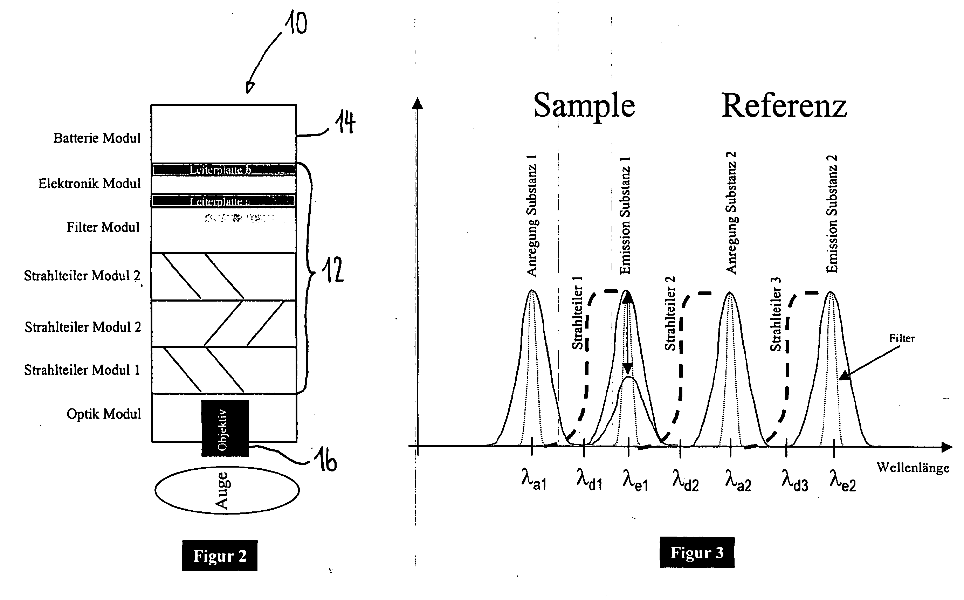

Figur 2 zeigt einen stark schematischen Längsschnitt durch das erfindungsgemäße Handmeßgerät 10 zur Veranschaulichung des mechanischen und optischen Aufbaus im Innern des Handmeßgeräts.

Das Handmeßgerät 10 umfaßt einen zentralen Abschnitt 12, an dessen einem Ende ein Batteriemodul 14 mit der zum Betreiben des Handmeßgeräts notwendigen Batterie und an dessen anderem Ende ein Optikmodul mit dem Objektiv 16 angeordnet sind. Der zentrale Abschnitt 12 des Handmeßgeräts 10 wiederum setzt sich aus mehreren Strahlteiler- und Filtermodulen sowie einem Elektronikmodul zusammen. Die Strahlteiler- und Filtermodule weisen vorteilhafterweise jeweils den gleichen Aufbau auf, der nachstehend noch ausführlich beschrieben ist.

In jedem der Strahlteilermodule 18 bis 22 sind jeweils ein dichroitischer Strahlteiler und ein Spiegel angeordnet. Eine weitere Möglichkeit ist durch die Verwendung eines Strahlteilerpaares gegeben, mit der aufgrund der Vorfilterung des zweiten Strahlteilers bessere Ergebnisse erzielt werden können. Der Winkel, unter dem Spiegel und Strahlteiler angeordnet sind, beträgt 45°. Die einzelnen Strahlteilermodule sind jeweils um 120° zueinander gedreht. Dadurch wird bei aufeinandergesetzten Strahlteilermodulen erreicht, daß die außerhalb der Zentralachse der Strahlteilermodule angeordneten Spiegel bei Draufsicht auf die Strahlteilermodule auf einem Kreis um die Zentralachse mit einem Winkelabstand von jeweils 120° verteilt angeordnet sind. Die dichroitischen Strahlteiler sind in der Zentralachse angeordnet. Aus dieser Anordnung resultiert eine konfokale Zusammenführung von auf die einzelnen Spiegel fallenden und von den Spiegeln auf die Strahlteiler reflektierten Lichtstrahlen zum Objektiv hin bzw. eine Auftrennung oder (je nach Wellenlänge) ein Durchlassen von durch das Objektiv in das Handmeßgerät fallendem Licht durch die Strahlteiler- und Spiegel-Anordnung.

Die lichtemittierenden und -detektierenden Elemente sind auf zwei Leiterplatten 28 und 30 des Elektronikmoduls 26 angeordnet. Die Anordnung der Elemente und der Abstand der beiden Leiterplatten 28, 30 sind derart gewählt, daß der Strahlenweg für emittiertes und detektiertes Licht jeweils gleich lang ist.

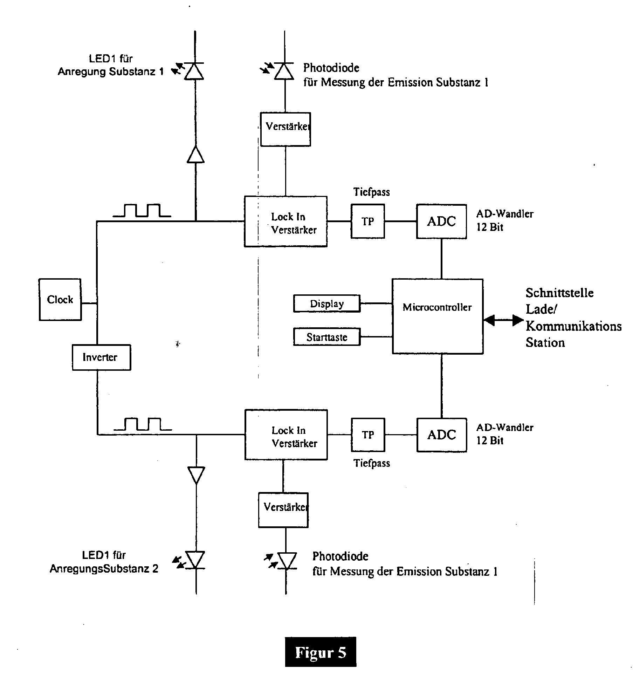

Wie aus der Darstellung der Figur 4 ersichtlich ist, ist auf den drei Strahlteilermodulen 18, 20, 22 noch ein Filtermodul 24 vorgesehen, in dem vier Filter angeordnet sind. Das Filtermodul mit den vier Filtern ist nicht zwingend notwendig, jedoch erfolgt durch die Filter eine Streulichtreduzierung (Bandpaßfilter), wodurch die Qualität der Messung verbessert wird. Zusätzlich oder alternativ können im Strahlengang Blenden oder Apparturen vorgesehen sein.

Es ergeben sich vier Strahlengänge:

| | |
|---|---|
| Strahlengang 1: | Lichtquelle LED 1 Auge, Anregungswellenlänge $\lambda_{a1}$ |
| Strahlengang 2: | Auge Sensor Photodiode 1, Emissionswellenlänge $\lambda_{e1}$ |
| Strahlengang 3: | Lichtquelle LED 2 Auge, Anregungswellenlänge $\lambda_{a2}$ |
| Strahlengang 4: | Auge Sensor Photodiode 2, Emissionswellenlänge $\lambda_{e2}$ |

Bei der ersten Substanz handelt es sich beispielsweise um Fluoreszein mit einer Anregungswellenlänge von $\lambda_{a1} \cong 488$ nm und einer Emissionswellenlänge von $\lambda_{e1} \cong 518$ nm. Bei der zweiten Substanz handelt es sich vorzugsweise um Rhodamin mit einer Anregungswellenlänge von $\lambda_{a2} \cong 560$ nm und einer Emissionswellenlänge von $\lambda_{e2} \cong 585$ nm.

Der optische Aufbau ist durch folgende Eigenschaften gekennzeichnet:

a) Die vier Strahlengänge sind konfokal angeordnet. Dadurch ergeben sich dieselben optischen Verhältnisse der einzelnen Strahlengänge bezüglich dem Winkel zwischen Meßgerät und Auge.

b) Die Abbildung der Lichtquellen auf das Auge und die Abbildung des emittierten Lichtes auf den Sensor ist für alle Strahlgänge identisch. Es ist somit nur ein Objektiv für die Abbildung erforderlich.

c) Die Trennung der einzelnen Strahlengänge erfolgt über die dichriotische Strahlteiler. Die dichriotischen Strahlteiler haben folgende Eigenschaften:

- Definierte Grenzwellenlänge $\lambda_d$
- Licht mit kürzerer Wellenlänge als die Grenzwellenlänge wird reflektiert.
- Licht mit größerer Wellenlänge als die Grenzwellenlänge wird durchgelassen.

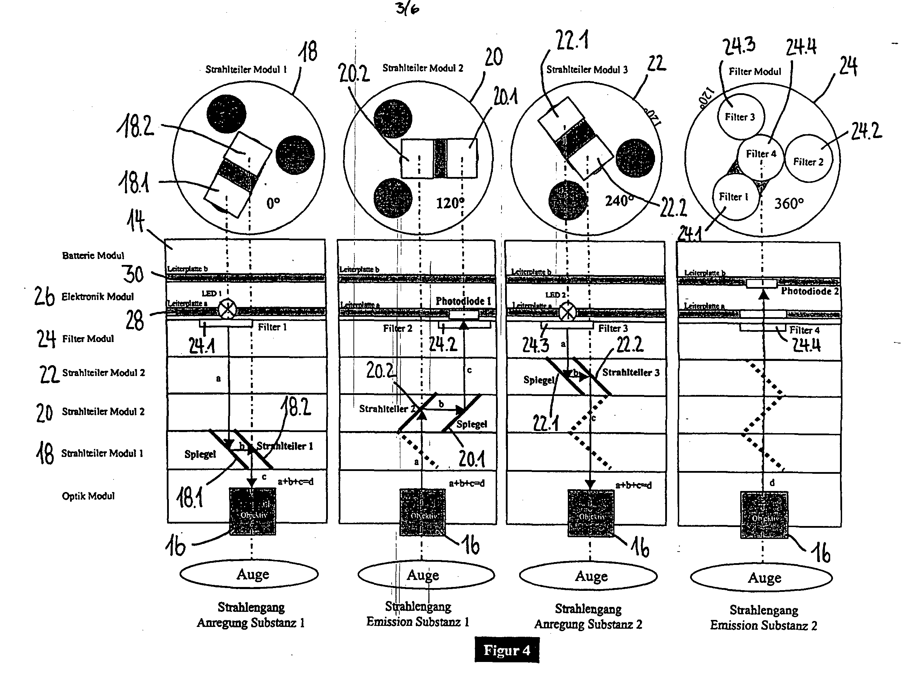

Es sind drei dichroitische Strahlteiler notwendig (vgl. Figur 3) :

$$\lambda_{a1} \ < \ \lambda_{d1} \ < \ \lambda_{e1}$$

$$\lambda_{e1} \ < \ \lambda_{d2} \ < \ \lambda_{a2}$$

$$\lambda_{a2} \ < \ \lambda_{d3} \ < \ \lambda_{e2}$$

Die dichroitischen Strahlteiler sind in einem Winkel von 45° zum Strahlengang positioniert. Die reflektierten Strahlen werden durch den jeweils zugeordneten Spiegel nochmals um 45° abgelenkt, so daß der Strahlengang jeweils parallel versetzt zum zentralen Strahl verläuft. Die Module mit den drei Strahlteilern und Spiegeln sind wie bereits erwähnt um jeweils 120° verdreht angeordnet. Somit befinden sich Lichtquellen und Sensor der Strahlengänge 1 bis 3 auf derselben Ebene. Im Strahlengang 4 wird kein dichroitischer Strahlteiler benötigt. Der Sensor im Strahlengang 4 wird aus diesem Grund um den Abstand Strahlteiler-Spiegel zurückversetzt. Der mechanische Aufbau für die Einheit Strahlteiler/Spiegel ist für drei Strahlengänge identisch. Sie unterscheiden sich lediglich durch die Grenzwellenlänge der eingesetzten Strahlteiler. Der optische Aufbau mit den einzelnen Strahlengängen ist in Figur 4 dargestellt.

d) Vor der Ebene der Sensoren und Lichtquellen kann optional ein Filter-Modul integriert werden zur spektralen Begrenzung der Anregungs- und Emissionsenergie.

e) Falls erforderlich werden im Strahlengang Blenden zur Reduzierung des Streulichts eingesetzt.

Im Handphotometer werden die Meßdaten ermittelt, berechnet und gespeichert. Es ist erforderlich, bei der Messung das Umgebungslicht auszufiltern. Aus diesem Grund werden die Lichtquellen alternierend mit einer definierten Taktrate angesteuert. Das Meßsignal am Sensor wird verstärkt. Das Nutzsignal wird ausgefiltert und über einen Analog-Digitalwandler in einen digitalen Wert gewandelt (vgl. Figur 5).

Aus dem Meßwert der Substanz 1 und dem Referenzwert aus der Substanz 2 wird die Konzentration des zu messenden Stoffes berechnet. Eine absolute Messung ist mit dem erfindungsgemäßen Meßgerät nicht möglich, da die Konzentration der fluoreszierenden Substanzen nicht konstant ist. Die Konzentration des zu messenden Stoffes in der Tränenflüssigkeit schwankt individuell sehr stark. Es ist deshalb notwendig, die wirkliche Konzentration des zu messenden Stoffes mit herkömmlichen Untersuchungsmethoden zu bestimmen und das Meßgerät auf diese Werte individuell zu kalibrieren. Die Kalibrierfaktoren werden im Handmeßgerät gespeichert und fließen mit in die Konzentrationsberechnung ein.

Die Messung wird durch eine Taste am Handmeßgerät gestartet. Der Meßwert wird an einer Anzeige (Display) des Handmeßgeräts angezeigt.

Im Meßgerät kann eine begrenzte Anzahl von Meßdaten gespeichert werden. Über eine standardisierte Schnittstelle kann das Meßgerät mit einem Datenerfassungssystem kommunizieren. Dies kann ein Standard-PC sein oder ein PDA (Personal Digital Assistent).

Das Datenerfassungs-System übernimmt die folgenden Funktionen:

- Speicherung der Meßdaten über einen langen Zeitraum.
- Auswertung der Meßdaten (Statistik)
- Graphische Darstellung
- Ausdrucken von Protokollen
- Senden der Kalibrierdaten zum Handmeßgerät
- Service/Wartung

Die Kommunikation zwischen Handmeßgerät und Datenerfassungssystem erfolgt in besonders bevorzugter Ausgestaltung über eine kombinierte Lade-Kommunikationsstation in der

- die Batterien geladen werden
- die Schnittstelle zum Datenerfassungssystem realisert wird.

Vorteilhafterweise umfaßt die Ladeeinheit einen eigenen integrierten Web-Server, so daß auf der Datenverarbeitungseinheit lediglich eine Browsersoftware benötigt wird.

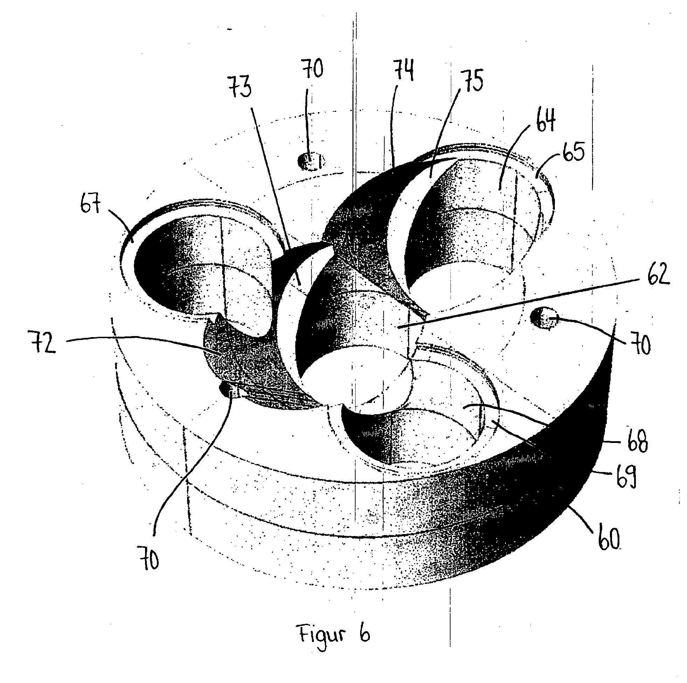

Figur 6 zeigt in perspektivischer Darstellung eine kreisscheibenförmige Platte 60, die das Basismodul für den Aufbau des erfindungsgemäßen Handmeßgeräts dient und entweder als Strahlteilermodul oder als Filtermodul einsetzbar ist.

Die Platte 60 weist mehrere Bohrungen und Ausnehmungen auf. Eine erste Bohrung 62 verläuft durch die Platte 60 koaxial zu deren Zentralachse (Rotationsachse). Rotationssymmetrisch um die erste Bohrung 62 sind drei weitere durchgehende Bohrungen 64, 66, 68 equidistant zur ersten Bohrung 62 und parallel zu dieser vorgesehen. Die Bohrungen 62, 64, 66, 68 weisen jeweils den gleichen Durchmesser auf. Bei mehreren übereinandergesetzten Platten bilden die einzelnen Bohrungen 62, 64, 66, 68 bei fluchtender Ausrichtung Kanäle, die im erfindungsgemäßen Handmeßgerät Strahlengangkanäle bilden. Zur äquidistanten und -angularen Ausrichtung sind zwischen den drei äußeren Bohrungen 64, 66, 68 noch kleinere Stiftbohrungen 70 vorgesehen, die ebenfalls jeweils einen Winkelabstand von 120° zueinander aufweisen und bei Aufeinandersetzen von einzelnen Platten 60 zur Ausrichtung und Festlegung durch Einsetzen von Stiften dienen.

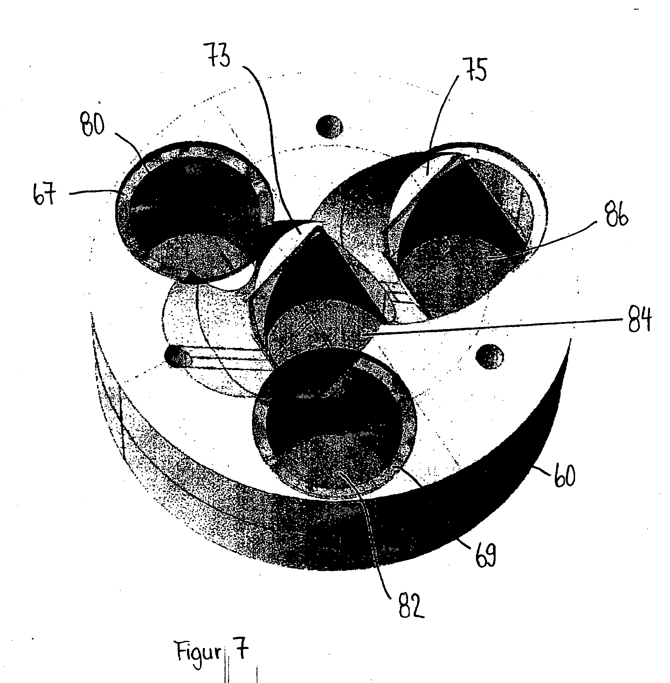

Die drei peripheren Bohrungen 64, 66, 68 weisen jeweils eine ringförmige Schulterausnehmung 65, 67, 69 auf, deren Durchmesser und Höhe einzulegenden Filterscheiben entspricht (vgl. Figur 7, in der eine Platte 60 mit zwei eingelegten Filterscheiben 80, 82 abgebildet ist).

Die zentrale Bohrung 62 und eine der umgebenden Bohrungen 64 weisen unter einem Winkel von 45° angeordnete zylinderförmige Ausnehmungen 72, 74 auf, deren Durchmesser dem Durchmesser bzw. den Randabmessungen von einzulegenden Spiegeln und Strahlteilern entspricht. Dieser Durchmesser ist größer als der Durchmesser der Strahlengangbohrungen 62, 64, so daß im Bereich des Übergangs zwischen den Ausnehmungen 72, 74 und den Bohrungen 62, 64 Schultern 73, 75 entstehen, die einen Neigungswinkel von 45° aufweisen und auf die die einzusetzenden Spiegel bzw. Strahlteiler aufgelegt werden (vgl. hierzu wiederum Figur 7, in der eine Platte 60 mit eingesetztem Strahlteiler 84 und eingesetztem Spiegel 86 eingesetzt sind). Die Ausnehmungen 72, 74 sind derart gestaltet, daß bei eingesetztem Spiegel und Strahlteiler ein senkrecht auf den Spiegel auftreffender Lichtstrahl um 90° auf den Strahlteiler hin abgelenkt wird. Der Strahlengang zwischen Spiegel und Strahlteiler ist dann ermöglicht, wenn durch die unter 45° ausgebildete Ausnehmung 74, die den Spiegel 86 aufnimmt, zwischen der zentralen Bohrung 62 und der unterhalb des Spiegels 86 zu liegen kommenden Bohrungen 64 ausreichend Plattenmaterial entfernt ist, daß ein Strahlendurchgang möglich ist.

Die erfindungsgemäße Platte 60, die das Grundmodul für ein erfindungsgemäßes Handmeßgerät bildet, kann einfach und kostengünstig in Massenfertigung mittels Drehen aus einem leichten und stabilen Werkstoff (beispielsweise Aluminium) aber auch aus einem Guß- oder Spritzteil hergestellt werden. Zum Aufbau eines erfindungsgemäßen Handmeßgeräts werden in drei derartige Platten Strahlteiler und Spiegel eingesetzt und die einzelnen Platten um jeweils 120° zueinander verdreht aufeinandergesetzt und mittels durch die Stiftbohrungen einzusetzender Stifte drehfest gesichert. Durch Einsetzen von Filterscheiben in die Schulterausnehmungen der einzelnen Bohrungen kann das gleiche Basismodul als Filtermodul verwendet werden.

Zusammenfassend wird somit eine Vorrichtung bereitgestellt, die insbesondere zur Messung körpereigener Stoffe durch optische Detektion am menschlichen Auge geeignet ist und die sich dadurch auszeichnet,

a) daß sie durch den kompakten optischen Aufbau und die geringe Stromaufnahme als mobiles Handmeßgerät mit Batteriebetrieb realisierbar ist,
b) daß im Handmeßgerät die komplette Meßwertaufnahme einschließlich Kalibrationsverfahren und ein Meßwertspeicher integriert ist,
c) daß in einer separaten Lade/Kommunikations-Station die Batterien geladen werden und gleichzeitig die Meßdaten in ein Datenverarbeitungssystem zur Weiterverarbeitung übertragen werden,
d) daß sie kostengünstig als Massenprodukt herstellbar ist,
e) daß keine optische Justage erforderlich ist,
f) daß die Fertigung der Module und die Endmontage der einzelnen Module automatisiert erfolgen kann,
g) daß die Anregungslichtquellen bezüglich Intensität und Wellenlänge unschädlich sind.
h) daß durch die konfokal angeordneten Strahlengänge die Meßwerte unempfindlich gegenüber Änderungen des Meßwinkels sind,
i) daß durch die alternierende getaktete Ansteuerung der Lichtquellen das Meßsignal gefiltert werden kann, und das Meßsignal somit weitestgehend unabhängig vom Umgebungslicht ist,
j) daß die Konzentration des körpereigenen Stoffes aus einem Meßsignal und einem Referenzsignal berechnet wird und somit der Abstand vom Meßgerät und die individuellen Eigenschaften des Auges keinen Einfluß auf das Meßsignal haben,
k) Kalibrierfaktoren in die Berechnung mit einfließen, so daß das Meßgerät auf die Meßwerte von konventionellen absoluten Meßmethoden kalibrierbar ist.

Des weiteren wird eine Ladeeinheit bereitgestellt, die sich dadurch auszeichnet,

a) daß beim Ladevorgang der Batterien des Handmeßgerätes eine Kommunikation mit einem Datenverarbeitungssystem über Standardschnittstellen möglich ist,
b) daß Meßdaten aus dem Handmeßgerät in ein Datenverarbeitungssystem übertragen werden,
c) daß Parameter und Kalibrierfaktoren vom Datenverarbeitungssystem in das Handmeßgerät übertragen werden.

Die Erfindung betrifft in einer Ausführungsform ein Meßsystem zur Messung der emittierten Lichtenergie fluoreszierender Substanzen im menschlichen Auge. Das Meßsystem ist ein Meßmittel in einem Verfahren in dem fluoreszierende Substanzen zur Messung der Konzentration körpereigener Stoffe in der Tränenflüssigkeit verwendet werden. Die Vorrichtung kann als batteriebetriebenes Handmeßgerät ausgeführt und kann vom Patienten ohne Fachkenntnisse zur medizinischen Überwachung eingesetzt werden. Das Meßsystem kann automatisiert in großen Stückzahlen gefertigt werden. Eine separate Batterieladestation mit integrierter Schnittstelle zu einem Datenverarbeitungssystem ermöglicht die Speicherung und Verwaltung der Meßdaten.

**Patentansprüche**

1. Basismodul zum modularen Aufbau einer optischen Messvorrichtung, das als kreisscheibenförmige Platte (60) ausgebildet ist, mit einer ersten, koaxial zu einer Zentralachse des Basismoduls (18, 20, 22) angeordneten Bohrung (62) und einer Anzahl von weiteren rotationssymmetrisch zu der ersten Bohrung (62) und parallel zu der Zentralachse des Basismoduls (18, 20, 22) angeordneten Bohrungen (64, 66, 68), wobei die erste Bohrung (62) zur Aufnahme eines Strahlteilers (84) und eine der weiteren Bohrungen (64, 66, 68) zur Aufnahme eines weiteren reflektierenden Elements (86) vorgesehen ist, wobei beide Bohrungen unter einem Winkel von 45° zur Zentralachse angeordnete zylinderförmige Ausnehmungen (72,74) aufweisen, so daß in Bereich des Übergangs zwischen den Ausnehmungen und den Bohrungen Schultern (73, 75) zur Aufnahme des Strahlteilers sowie des reflektierenden Elementes entstehen, wobei durch die Ausnehmungen ausreichend Plattenmaterial zwischen den Bohrungen entfernt ist, so daß ein Strahlendurchgang möglich ist, und bei dem der Strahlteiler (84) sowie das weitere reflektierende Element (86) einen Neigungswinkel von 45° aufweisen, so dass ein senkrecht auf das Basismodul (18, 20, 22) auftreffender Lichtstrahl entweder durch den Strahlteiler (84) auf das reflektierende Element (86) hin oder durch das reflektierende Element (84) auf den Strahlteiler hin um 90° abgelenkt wird.

2. Basismodul nach Anspruch 1, bei dem das weitere reflektierende Element (84, 86) ein Strahlteiler (84) ist.

3. Basismodul nach Anspruch 1, bei dem das weitere reflektierende Element (84, 86) ein Spiegel (86) ist.

4. Basismodul nach einem der Ansprüche 1 bis 3, bei dem der Strahlteiler (84) und das weitere reflektierende Element (84, 86) im wesentlichen parallel zueinander angeordnet sind.

5. Basismodul nach einem der Ansprüche 1 bis 4, bei der die zentrale Bohrung (62) und mindestens eine der weiteren Bohrungen (64, 66, 68) unter einem Winkel von 45° angeordnete zylinderförmige Ausnehmungen (65, 67, 69) aufweisen, deren Durchmesser den Randabmessungen von einzulegenden Strahlteilern (84) und reflektierenden Elementen (84, 86) entspricht.

6. Basismodul nach einem der Ansprüche 1 bis 5, bei dem die weiteren Bohrungen (64, 66, 68) equidistant zueinander angeordnet sind.

7. Basismodul nach einem der Ansprüche 1 bis 6, bei dem die weiteren Bohrungen (64, 66, 68) und die zentrale Bohrung (62) den gleichen Durchmesser aufweisen.

8. Basismodul nach einem der Ansprüche 1 bis 7, bei dem in an benachbarte Basismodule (18, 20, 22) angrenzenden Flächen Stiftbohrungen (70) vorgesehen sind.

9. Vorrichtung zur simultanen Detektion von Strahlungen unterschiedlicher Wellenlänge mit einer Anzahl von übereinander angeordneten Basismodulen (18, 20, 22) nach einem der Ansprüche 1 bis 8, einem Optikmodul mit einem Objektiv (16) und einem Elektronikmodul (26) mit lichtdetektierenden Elementen, wobei in den Basismodulen (18, 20, 22) jeweils eine Einrichtung (84, 86) zum Reflektieren bzw. Umleiten von Strahlung eines vorbestimmbaren Wellenlängenbereichs vorgesehen ist und die lichtdetektierenden Elemente jeweils mit einer der Einrichtungen (84, 86) korrespondieren.

10. Vorrichtung nach Anspruch 9, bei der die Basismodule (18, 20, 22) um einen Winkel zueinander gedreht angeordnet sind, so daß die außerhalb einer Zentralachse der Basismodule angeordneten Einrichtungen (84, 86) auf einem Kreis um die Zentralachse verteilt angeordnet sind.

11. Vorrichtung nach Anspruch 9 oder 10, bei der im Elektronikmodul (26) mindestens ein lichtemittierendes Element mit definierter Wellenlänge vorgesehen ist, so daß Licht dieser definierten Wellenlänge zur Anregung einer Substanz aus der Vorrichtung austritt.

12. Vorrichtung nach Anspruch 11, bei der die lichtemittierenden und die lichtdetektierenden Elemente auf Leiterplatten (28, 30) angeordnet sind.

13. Vorrichtung nach einem der Ansprüche 9 bis 12, bei der zusätzlich ein Filtermodul (24) vorgesehen ist.

14. Vorrichtung nach einem der Ansprüche 9 bis 13, bei der Blenden vorgesehen sind.

**15.** Verfahren zum Einstellen einer Vorrichtung (10) nach einem der Ansprüche 9 bis 14, bei dem zur Einstellung der Vorrichtung (10) eine Fokussierung durchgeführt wird.

**16.** Verfahren nach Anspruch 15, bei dem durch Ändern des Abstands zwischen Vorrichtung (10) und des zu vermessenden Objekts die Einstellung vorgenommen wird, indem während einer Änderung des Abstands gemessen wird und anhand des Verlaufs der gemessenen Daten in Abhängigkeit des Abstands der geeignete Abstand ermittelt wird.

**17.** Verwendung einer Vorrichtung (10) nach einem der Ansprüche 9 bis 14 zur Messung körpereigener Stoffe durch Detektion im menschlichen Auge.


**Claims**

**1.** Basic module for the modular construction of an optical measuring device which is constructed as a circular disc-shaped plate (60), having a first bore (62) arranged coaxially with a central axis of the basic module (18, 20, 22) and a number of further bores (64, 66, 68) arranged rotationally symmetrically to the first bore (62) and parallel to the central axis of the basic module (18, 20, 22), the first bore (62) being provided to receive a beam splitter (84) and one of the further bores (64, 66, 68) being provided to receive a further reflecting element (86), both bores having cylindrical recesses (72, 74) arranged at an angle of 45° to the central axis, so that in the region of the transition between the recesses and the bores shoulders (73, 75) are formed, to accommodate the beam splitter and the reflecting element, wherein the provision of the recesses has caused sufficient plate material to be removed between the bores to allow a beam to pass through, and wherein the beam splitter (84) and the further reflecting element (86) are at an angle of 45°, so that a light beam striking the base module (18, 20, 22) perpendicularly is deflected through 90° either by the beam splitter (84) onto the reflecting element (86) or by the reflecting element (84) onto the beam splitter.

**2.** Basic module according to claim 1, wherein the further reflecting element (84, 86) is a beam splitter (84).

**3.** Basic module according to claim 1, wherein the further reflecting element (84, 86) is a mirror (86).

**4.** Basic module according to one of claims 1 to 3, wherein the beam splitter (84) and the further reflecting element (84, 86) are arranged substantially parallel to one another.

**5.** Basic module according to one of claims 1 to 4, wherein the central bore (62) and at least one of the further bores (64, 66, 68) have cylindrical recesses (65, 67, 69) arranged at an angle of 45°, the diameter of which corresponds to the edge dimensions of beam splitters (84) and reflecting elements (84, 86) to be placed therein.

**6.** Basic module according to one of claims 1 to 5, wherein the further bores (64, 66, 68) are arranged equidistant from one another.

**7.** Basic module according to one of claims 1 to 6, wherein the further bores (64, 66, 68) and the central bore (62) have the same diameter.

**8.** Basic module according to one of claims 1 to 7, wherein pin bores (70) are provided in adjoining surfaces on adjacent basic modules (18, 20, 22).

**9.** Device for simultaneously detecting radiation of different wavelengths having a number of basic modules (18, 20, 22) arranged one above the other, according to one of claims 1 to 8, an optical module having an objective (16) and an electronic module (26) with light-detecting elements, wherein in each of the basic modules (18, 20, 22) a device (84, 86) is provided for reflecting or diverting radiation in a predeterminable wavelength range and the light-detecting elements each correspond to one of the devices (84, 86).

**10.** Device according to claim 9, wherein the basic modules (18, 20, 22) are arranged in a position rotated at an angle to one another, so that the devices (84, 86) arranged outside a central axis of the basic modules are distributed in a circle about the central axis.

**11.** Device according to claim 9 or 10, wherein in the electronic module (26) is provided at least one light-emitting element of a defined wavelength, so that light of this defined wavelength for exciting a substance is emitted from the device.

12. Device according to claim 11, wherein the light-emitting and light-detecting elements are arranged on printed circuit boards (28, 30).

13. Device according to one of claims 9 to 12, wherein additionally a filter module (24) is provided.

14. Device according to one of claims 9 to 13, wherein shutters are provided.

15. Method of adjusting a device (10) according to one of claims 9 to 14, wherein focussing is carried out in order to adjust the device (10).

16. Method according to claim 15, wherein by varying the distance between the device (10) and the object which is to be measured, the adjustment is made by performing the measurement while varying the spacing and the appropriate distance is determined by means of the changes in the data measured as a function of the distance.

17. Use of a device (10) according to one of claims 9 to 14, for measuring bodily substances by detection in the human eye.


**Revendications**

1. Module de base pour la réalisation modulaire d'un dispositif de mesurage optique, qui est réalisé comme un plateau (60) en forme de disque circulaire, doté d'un premier perçage (62) agencé coaxialement à un axe central du module de base (18, 20, 22), et d'un certain nombre d'autres perçages (64, 66, 68) agencés avec symétrie de révolution par rapport au premier perçage (62) et parallèles à l'axe central du module de base (18, 20, 22), le premier perçage (62) étant prévu pour recevoir un subdiviseur de faisceau (84) et l'un des autres perçages (64, 66, 68) étant prévu pour recevoir un autre élément réfléchissant (86), les deux perçages présentant des évidements (72, 74) de forme cylindrique agencés sous un angle de 45° par rapport à l'axe central, de sorte que dans la zone de la transition entre les évidements et les perçages se forment des épaulements (73, 75) pour la réception du subdiviseur de rayonnement et de l'élément réfléchissant, et une quantité suffisante de matériau de plaque est enlevée entre les perçages du fait des évidements pour permettre un passage du rayonnement, et dans lequel le subdiviseur de rayonnement (84) ainsi que l'autre élément réfléchissant (86) présentent un angle d'inclinaison de 45°, de sorte qu'un rayon lumineux qui tombe perpendiculairement sur le module de base (18, 20, 22) est dévié de 90° soit par le subdiviseur de rayonnement (84) vers l'élément réfléchissant (86) soit par l'élément réfléchissant (84) vers le subdiviseur de rayonnement.

2. Module de base selon la revendication 1, dans lequel l'autre élément réfléchissant (84, 86) est un subdiviseur de rayonnement (84).

3. Module de base selon la revendication 1, dans lequel l'autre élément réfléchissant (84, 86) est un miroir (86).

4. Module de base selon l'une des revendications 1 à 3, dans lequel le subdiviseur de rayonnement (84) et l'autre élément réfléchissant (84, 86) sont agencés sensiblement parallèlement l'un à l'autre.

5. Module de base selon l'une des revendications 1 à 4, dans lequel le perçage central (62) et l'un au moins des autres perçages (64, 66, 68) présentent des évidements (65, 67, 69) de forme cylindrique agencés sous un angle de 45°, dont le diamètre correspond aux dimensions au bord des subdiviseurs de rayonnement (84) et des éléments réfléchissants (84, 86) à insérer.

6. Module de base selon l'une des revendications 1 à 5, dans lequel les autres perçages (64, 66, 68) sont agencés équidistants les uns des autres.

7. Module de base selon l'une des revendications 1 à 6, dans lequel les autres perçages (64, 66, 68) et le perçage central (62) ont le même diamètre.

8. Module de base selon l'une des revendications 1 à 7, dans lequel des perçages pour goupille (70) sont prévus dans des surfaces adjacentes à des modules de base voisins (18, 20, 22).

**9.** Dispositif pour la détection simultanée de rayonnement à longueurs d'ondes différentes comprenant un certain nombre de modules de base (18, 20, 22), agencés les uns au-dessus des autres, selon l'une des revendications 1 à 8, un module optique avec un objectif (16) et un module électronique (26) avec des éléments détecteurs de lumière, dans lequel, dans les modules de base (18, 20, 22), il est prévu respectivement un système (84, 86) pour réfléchir ou pour dévier le rayonnement d'une plage de longueur d'onde prédéterminée, et les éléments détecteurs de lumière correspondent chacun à l'un des systèmes (84, 86).

**10.** Dispositif selon la revendication 9, dans lequel les modules de base (18, 20, 22) sont tournés les uns par rapport aux autres d'un angle tel que les systèmes (84, 86) agencés à l'extérieur d'un axe central des modules de base sont agencés de façon répartie sur un cercle autour de l'axe central.

**11.** Dispositif selon l'une des revendications 9 ou 10, dans lequel, dans le module électronique (26), est prévu au mains un élément émetteur de lumière avec une longueur d'onde définie, de sorte que la lumière avec cette longueur d'onde définie sort du dispositif pour exciter une substance.

**12.** Dispositif selon la revendication 11, dans lequel les éléments émetteurs de lumière et les éléments détecteurs de lumière sont agencés sur des plaques à circuits imprimés (28, 30).

**13.** Dispositif selon l'une des revendications 9 à 12, dans lequel il est prévu en supplément un module de filtre (24).

**14.** Dispositif selon l'une des revendications 9 à 13, dans lequel il est prévu des diaphragmes.

**15.** Procédé pour régler un dispositif (10) selon l'une des revendications 9 à 14, dans lequel on exécute une focalisation pour le réglage du dispositif (10).

**16.** Procédé selon la revendication 15, dans lequel, par modification de la distance entre le dispositif (10) et l'objet à mesurer on exécute le réglage en mesurant pendant une modification de la distance et l'on détermine la distance appropriée à l'aide de l'évolution des données mesurées en dépendance de la distance.

**17.** Utilisation d'un dispositif (10) selon l'une des revendications 9 à 14 pour mesurer des produits corporels par détection dans l'oeil humain.

Handmessgerät mit Batteriebetrieb

Datenverarbeitungssystem

(Standard-PC, Labtop, PDA )

Lade/Kommunikations-Station

Standard Kommunikationsschnittstelle
( RS232, Netzwerk, Bluetooth,
Infrarot, USB )

Figur 1

**Figur 3**

Referenz

Sample

Wellenlänge

Filter

Emission Substanz 2
Strahlteiler 3
Anregung Substanz 2
Strahlteiler 2
Emission Substanz 1
Strahlteiler 1
Anregung Substanz 1

$\lambda_{e2}$ $\lambda_{d3}$ $\lambda_{a2}$ $\lambda_{d2}$ $\lambda_{e1}$ $\lambda_{d1}$ $\lambda_{a1}$

**Figur 2**

Batterie Modul
Elektronik Modul
Filter Modul
Strahlteiler Modul 2
Strahlteiler Modul 2
Strahlteiler Modul 1
Optik Modul

Leiterplatte b
Leiterplatte a

Objektiv

Auge

10
14
12
16

13

Figur 4

Figur 5

Figur 6

EP 1 379 845 B1

Figur 7

EP 1 379 845 B1